# EUROPÄISCHE PATENTSCHRIFT

(11) **EP 1 216 657 B1**
(45) Veröffentlichungstag und Bekanntmachung des Hinweises auf die Patenterteilung: **29.06.2005**
(21) Anmeldenummer: 01129499.8
(22) Anmeldetag: 11.12.2001
(51) Int. Cl.: A61B 5/103

(54) **Prüfgerät zur Überwachung der beim Gehen auftretenden Druckbelastung des Fusses**
Device for monitoring pressure applied on foot during walking
Dispositif pour surveiller la pression appliquée sur le pied durant la marche

(30) Priorität: 19.12.2000 DE 20021422 U
(43) Veröffentlichungstag der Anmeldung: 26.06.2002
(73) Patentinhaber: Bechmann, Peter, Dipl.-Ing. (FH), D-82487 Oberammergau (DE)
(72) Erfinder: Bechmann, Peter, Dipl.-Ing. (FH), D-82487 Oberammergau (DE)
(74) Vertreter: Müller, Frank Peter, Dipl.-Ing.

(56) Entgegenhaltungen:
- DE-A- 19 625 474
- US-A- 2 850 937

## Beschreibung

Die Erfindung bezieht sich auf ein Prüfgerät zur Überwachung der beim Gehen auftretenden Druckbelastung des Fußes, die durch das Körpergewicht ausgeübt wird.

Bei der Therapie von Beinfrakturen sowie nach orthopädischen Operationen bsp. der Hüfte ist es für den Heilprozeß wichtig, daß die Belastung des Fußes nur mit einem allmählich fortschreitenden Anteil des Körpergewichts des Patienten stattfindet. Es werden dafür bestimmte Vorgaben erteilt, die mit einem minimalen Anteil des Körpergewichts als einer anfänglichen maximalen Druckbelastung beginnen. Diese Druckbelastung wird dann schrittweise erhöht, bis schließlich das gesamte Körpergewicht als Druckbelastung auf den Fuß am Ende des Heilungsprozesses abgeleitet werden kann. Nur mit einer strikten Einhaltung der einzelnen Stufen einer solchen fortschreitenden Druckbelastung kann somit ein optimaler Heilungsprozeß erwartet werden. Es ist deshalb auch entsprechend wichtig, daß eine kontinuierliche Überwachung der Druckbelastung des Fußes beim Gehen ausgeübt wird.

Aus der DE 41 00 834 A1 ist ein Prüfgerät bekannt, welches mit einer elastischen, unter einem Fuß anzuordnenden und mit einem Druckmittel, nämlich mit Luft atmosphärischen Druckes, gefüllten Druckmittelkammer ausgebildet ist. Diese Druckmittelkammer ist als ein Drucksensor in eine Schuhsohle eingearbeitet und über eine flexible Leitung mit einem analogen Anzeigeinstrument verbunden, welches eine Meßelektronik und Signallampen aufweist, mit denen eine richtige oder unzulässig hohe Belastung des Fußes angezeigt wird.

Aus der US 6 031 463 ist ein vergleichbares Prüfgerät bekannt, bei welchem als Drucksensor ein dabei mit einem Fluid gefülltes, elastisch deformierbares Fußkissen vorgesehen ist, das über eine Fluidleitung mit einer als Druckwächter dienenden Blase verbunden ist, welche in Abhängigkeit von dem über die Fluidleitung vermittelten Fluiddruck einen Signalgeber in der Ausbildung einer Klickfeder betätigen kann. Die Klickfeder vermittelt mit einem hörbaren Klickgeräusch eine akustische Anzeige bei der Überschreitung einer vorbestimmten maximalen Druckbelastung des Fußes. Die Klickfeder ist im übrigen innerhalb eines Gehäuses angeordnet. Sie ist gegen einen durch die Blase des Druckwächters betätigbaren Betätigungshebel verspannt, der die Klickfeder in Abhängigkeit von dem an dem Druckwächter abgetasteten Fluiddruck für die Abgabe des Klickgeräusches aus einer Ausgangslage heraus bewegen kann, in welche die Klickfeder wieder zurückgeführt wird, sobald die mit dem Druckwächter abgetastete Druckbelastung des Fußes wieder aufgehoben wird.

Aus der DE 196 25 474 A ist ein Prüfgerät gemäß dem Oberbegriff des selbständigen Patentanspruches 1 bekannt, bei dem die Scheibenfeder als eine tellerartige Metall-Feder ausgebildet ist, die mit einem Randbereich auf einem ringförmigen Rand einer aus einem härteren Kunststoff bestehenden unteren Platte aufliegt und mit einem mittigen gewölbten Bereich eine Öffnung in dieser unteren Platte überdeckt. Mit diesem gewölbten Bereich wird ein Schnappteil erhalten, der bei Ausübung einer Druckkraft von oben her unter Vermittlung einer der Ferse eines menschlichen Fußes angepassten Standfläche zum Umschnappen gebracht wird und dabei ein Klickgeräusch erzeugt, das als eine akustische Anzeige mithin bei der Überschreitung einer vorbestimmten maximalen Druckbelastung des Fußes dient. Es ist noch angegeben, dass die tellerartigen Metall-Federn für verschieden hohe Belastungen ausgelegt sein können.

Die US 2 850 937 offenbart eine quadratische Scheibenfeder, die in der Mitte ein Loch aufweist und unter einer vorbestimmten maximalen Druckbelastung aus einer Ausgangslage in einen Überwachungszustand umschnappt und bei einer Druckentlastung wieder in die Ausgangslage zurückfedert.

Der Erfindung liegt die Aufgabe zugrunde, ein Prüfgerät bereitzustellen, welches für die Überwachung der beim Gehen auftretenden Druckbelastung des Fußes eine größere Sicherheit erwarten lässt unter Einbeziehung auch einer Einhaltung der einzelnen Stufen, die im Rahmen einer therapeutischen Behandlung mit einer fortschreitenden Druckbelastung des Fußes vorgegeben werden.

Diese Aufgabe wird erfindungsgemäß mit einem Prüfgerät gelöst, welches mit den Merkmalen des selbständigen Patentanspruches 1 ausgebildet ist.

Bei dem erfindungsgemäßen Prüfgerät ist somit die Vorkehrung getroffen, daß immer dann, wenn die maximale Druckbelastung des Fußes erreicht wird, die mit der als Drucksensor verwendeten Scheibenfeder vorbestimmt ist, ein hörbares und gleichzeitig auch an der Ferse spürbares Umschnappen des Schnappteils der Scheibenfeder stattfindet. Die Spürbarkeit des Umschnappens wird durch die Druckplatten verstärkt und kann deshalb auch von älteren Personen sofort bemerkt werden, sodaß dann eine augenblickliche Druckentlastung des Fußes vorgenommen werden kann. Das erfindungsgemäße Prüfgerät wird daher eine sehr zuverlässige Überwachung der beim Gehen auftretenden Druckbelastung des Fußes gewährleisten und ergibt im Vergleich zu den bekannten Geräten eine sehr kostengünstige Variante, die wegen des Verzichts auf besondere Fluid- bzw. Signalleitungen auch wesentlich einfacher und weniger störanfällig unterhalten werden kann.

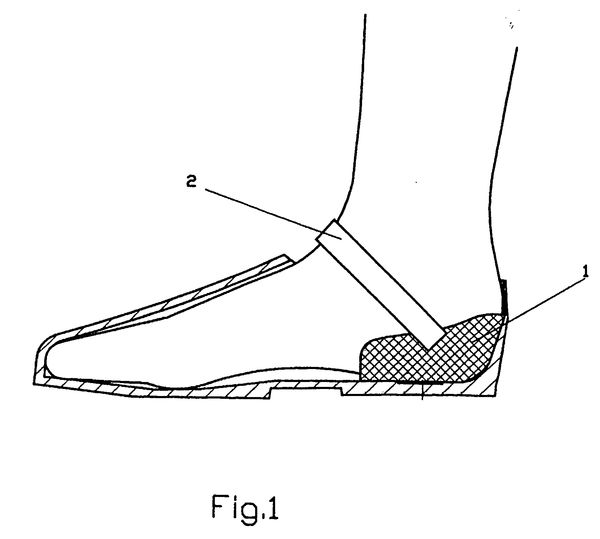

Weitere zweckmäßige und vorteilhafte Ausbildungen des erfindungsgemäßen Prüfgerätes sind in den einzelnen Ansprüchen angegeben.
Ein Ausführungsbeispiel des erfindungsgemäßen Prüfgerätes ist in der Zeichnung schematisch dargestellt und wird nachfolgend näher beschrieben. Es zeigt
- Fig. 1: eine Seitenansicht eines Fußes mit einer Darstellung des Prüfgerätes,

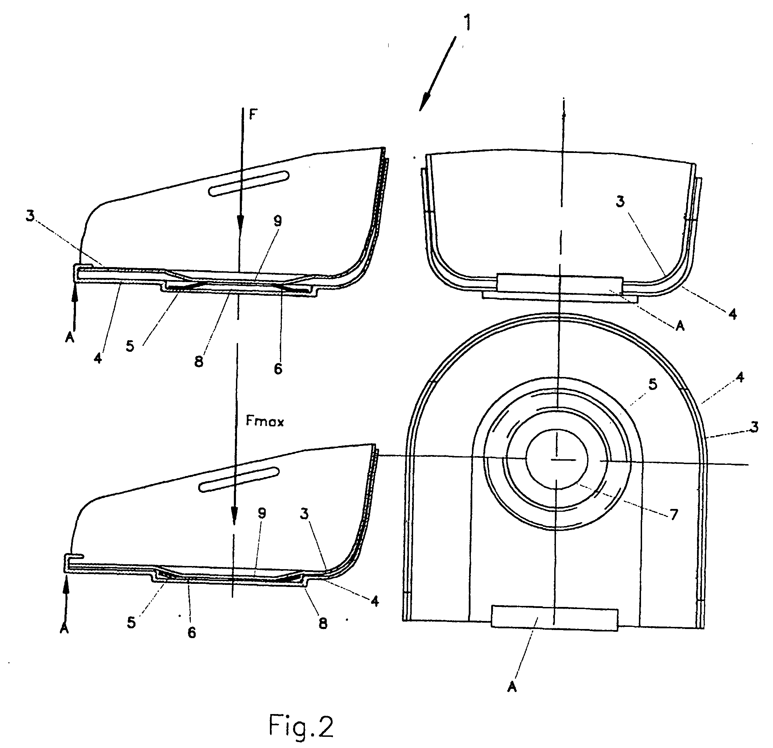

- Fig. 2: verschiedenen Ansichten des Prüfgerätes, wobei die als Drucksensor verwendete Scheibenfeder in der unbelasteten Ausgangslage und in dem umgeschnappten Überwachungszustand ihres Schnappteils gezeigt ist,
- Fig. 3: eine Schemadarstellung zur Veranschaulichung der beiden Funktionslagen des Schnappteils der Scheibenfeder und
- Fig. 4: eine Seitenansicht des Prüfgerätes im aufgeklappten Zustand.

Das zur Überwachung der beim Gehen auftretenden Druckbelastung des Fußes vorgesehene Prüfgerät 1 wird mittels eines Riemens 2 an der Ferse befestigt. Das Prüfgerät weist zwei nach Art eines Fersenbettes schalenförmig ausgebildete Druckplatten 3 und 4 auf, zwischen welchen eine kreisringförmige Scheibenfeder 5 angeordnet wird, die eine konische Erhebung 6 aufweist, welche das zentrale Loch 7 der Feder umgibt. Diese konische Erhebung 6 stellt einen zentralen Schnappteil der Scheibenfeder 5 dar, welche einen eigentlichen Drucksensor des Prüfgerätes bildet, mit dem die Druckbelastung des Fußes abgetastet wird.

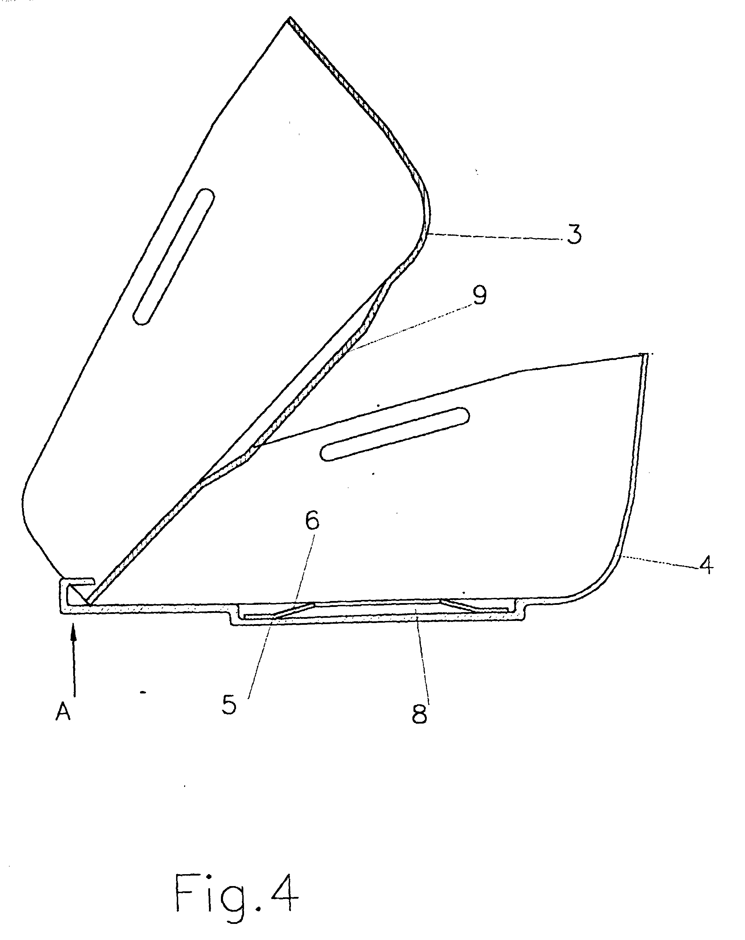

Die beiden schalenförmig ausgebildeten Druckplatten 3 und 4 sind an einem gemeinsamen Schwenklager A zusammengefaßt, sodaß sie um dieses Schwenklager gemäß der Darstellung in Fig. 4 aufgeklappt werden können. In dem aufgeklappten Zustand kann dann die Scheibenfeder in eine Vertiefung 8 der einen Druckplatte 4 so eingelegt werden, daß die konische Erhebung ihres Schnappteils nach oben ausgerichtet ist. Die zweite Druckplatte 3 wird dann zur Bereitstellung des Prüfgerätes um das Schwenklager A verschwenkt, bis ein an ihr ausgebildeter erhabener Bereich 9 zur Anlage an dem Schnappteil der Scheibenfeder 5 kommt. Das Prüfgerät ist dann mit der eingelegten Scheibenfeder fertig zur Befestigung an der Ferse eines Fußes, bei welchem die Druckbelastung durch das Körpergewicht beim Gehen überwacht werden soll.

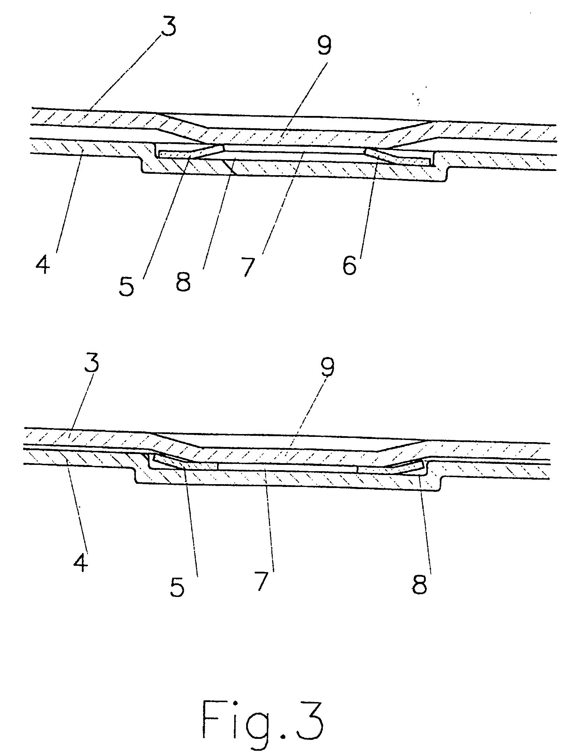

Wenn mit der Federhärte bzw. Federkennlinie der Scheibenfeder eine für die Therapie einer Beinfraktur zulässige maximale Druckbelastung von anfänglich nur etwa 20 kg berücksichtigt wird, dann nimmt der Schnappteil der Scheibenfeder bis zum Erreichen dieses Grenzwertes der Druckbelastung die in Fig. 3 oben dargestellte Ausgangslage ein. Sobald diese anfängliche maximale Druckbelastung erreicht und überschritten wird, dann wird die konische Erhebung 6 der Scheibenfeder aus der Ausgangslage in einen Überwachungszustand umgeschnappt, bei welchem somit durch den erhabenen Bereich 9 der einen Druckplatte 3 die konische Erhebung in die Vertiefung 8 der zweiten Druckplatte 4 gedrückt und gleichzeitig der angrenzende Ringteil der Scheibenfeder 5 nach oben umgeschnappt wird. Bei diesem Umschnappen entsteht ein Schnappgeräusch und gleichzeitig ein spürbarer Rückstoß an der Ferse des Patienten. Dadurch soll eine Druckentlastung des Fußes ausgelöst werden, die dann wieder ein Zurückschnappen des Schnappteils der Scheibenfeder in die Ausgangslage bewirkt, also wieder ein Lösen der konischen Erhebung 6 aus der Vertiefung 8 der Druckplatte, das von einem Rückschnappen des angrenzenden Ringteils der Scheibenfeder in die anfängliche Ausgangslage begleitet wird. Wenn bei der laufenden Therapie auf eine höhere maximale Druckbelastung übergewechselt werden kann, dann kann die nächste Stufe der Druckbelastung mit einem Austausch der Scheibenfeder gegen eine Feder überwacht werden, die dann eine geänderte Federhärte bzw. Federkennlinie mit einer Anpassung an diese höhere Stufe einer maximalen Druckbelastung aufweist. Wenn somit das Prüfgerät mit einzelnen Scheibenfedern ausgerüstet wird, die entsprechend unterschiedliche Federhärten bzw. Federkennlinien aufweisen, dann ist es damit möglich, die Überwachung eines Heilungsprozesses nach ärztlichen Vorgaben für eine stufenweise erhöhte Druckbelastung des therapierten Fußes sicher zu überwachen. Dabei können die Scheibenfedern mit einer unterschiedlichen Federhärte bzw. Federkennlinie zur Vereinfachung der Handhabung des Prüfgerätes auch mit unterschiedlichen Farben gekennzeichnet sein. Auch ist denkbar, daß die höheren Stufen der Druckbelastung mit einer geschichteten Anordnung von Scheibenfedern einer einheitlichen Federhärte bzw. Federkennlinie vorgegeben werden.

## Patentansprüche

1. Prüfgerät (1) zur Überwachung der beim Gehen auftretenden Druckbelastung des Fußes, die durch das Körpergewicht ausgeübt wird, mit einem Drucksensor, welcher für eine Prüfanzeige der Ferse des zu überwachenden Fußes unterlegt wird, wobei der Drucksensor mit einer Scheibenfeder (5) ausgebildet ist, die einen Schnappteil (6) aufweist, der unter einer vorbestimmten maximalen Druckbelastung des Fußes aus einer Ausgangslage in einen Überwachungszustand hörbar und an der Ferse spürbar umschnappt und der bei einer Druckentlastung des Fußes wieder in die Ausgangslage zurückfedert,
**dadurch gekennzeichnet, dass** die Scheibenfeder (5) kreisringförmig ausgebildet und der Schnappteil als eine konische Erhebung (6) geformt ist, welche das zentrale Loch (7) der Scheibenfeder (5) umgibt und den angrenzenden Ringteil der Scheibenfeder in den Überwachungszustand umschnappen lässt, und dass das Prüfgerät zwei als Schuheinlage ausgebildete Druckplatten (3,4) aufweist, zwischen welchen die kreisringförmige Scheibenfeder angeordnet ist.

2. Prüfgerät nach Anspruch 1, **dadurch gekennzeichnet, dass** die eine Druckplatte (3) als Fersenauflage benutzt wird und einen zur Anlage an dem Schnappteil (6) der kreisringförmigen Scheibenfeder (5) vorgesehenen erhabenen Bereich (9) aufweist.

3. Prüfgerät nach Anspruch 2, **dadurch gekennzeichnet, dass** die kreisringförmige Scheibenfeder (5) innerhalb einer mit dem erhabenen Bereich (9) der einen Druckplatte (3) fluchtenden Vertiefung (8) der zweiten Druckplatte (4) angeordnet ist, wobei mit der Vertiefung (8) der umgeschnappte Überwachungszustand des Schnappteils (6) der Scheibenfeder (5) begrenzt wird.

4. Prüfgerät nach Anspruch 2 oder 3, **dadurch gekennzeichnet, daß** die beiden Druckplatten (3, 4) nach Art eines Fersenbettes schalenförmig ausgebildet sind.

5. Prüfgerät nach einem der Ansprüche 2 bis 4, **dadurch gekennzeichnet, daß** die beiden Druckplatten (3, 4) um ein gemeinsames Schwenklager (A) relativ zueinander beweglich sind.

6. Prüfgerät nach Anspruch 5, **dadurch gekennzeichnet, daß** die beiden Druckplatten (3, 4) durch das gemeinsame Schwenklager (A) für eine gemeinsame Aufnahme der kreisringförmigen Scheibenfeder (5) ausgebildet sind.

7. Prüfgerät nach einem der Ansprüche 1 bis 6, **dadurch gekennzeichnet, daß** unterschiedliche maximale Druckbelastungen des Schnappteils (6) mit einer angepaßten unterschiedlichen Federhärte bzw. Federkennlinie von einzelnen kreisringförmigen Scheibenfedern (5) berücksichtigt sind.

8. Prüfgerät nach einem der Ansprüche 1 bis 6, **dadurch gekennzeichnet, daß** unterschiedliche maximale Druckbelastungen mit einer geschichteten Mehrfachanordnung von kreisringförmigen Scheibenfedern (5) berücksichtigt sind, die alle eine gleiche Federhärte bzw. Federkennlinie ihres Schnappteils (6) aufweisen.

## Claims

1. Device (1) for monitoring the load exerted by the body's weight onto the foot during walking, with a pressure sensor underlying the heel of the foot to be monitored for emitting a control signal, the pressure sensor being provided with a spring washer (5) having a snap element (6) which, at a predetermined maximum load exerted on the foot, audibly and at the heel noticeably snaps over from a starting position into a monitoring condition, and which upon load relief of the foot resiliently returns to the starting position, **characterized in that** the spring washer (5) is annulus-shaped and the snap element is designed as a conical elevation (6) surrounding the central hole (7) of the spring washer (5) and causing the adjacent ring portion of the spring washer to snap over into the monitoring condition, and that the monitoring device is provided with two shoe-pad like pressure plates (3, 4) with the annular-shaped spring washer located between them.

2. Monitoring device according to claim 1, **characterized in that** said one pressure plate (3) is used as a heel support and has a prominent area (9) for resting against the snap element (6) of the annular-shaped spring washer (5).

3. Monitoring device according to claim 2, **characterized in that** the annular-shaped spring washer (5) is located inside a recess (8) of the second pressure plate (4), in alignment with the prominent area (9) of said one pressure plate (3), whereas the snapped-over monitoring condition of the snap element (6) of the spring washer (5) is limited by the recess (8).

4. Monitoring device according to claims 2 or 3, **characterized in that** said two pressure plates (3, 4) are designed as a shell-like heel bed.

5. Monitoring device according to any one of claims 2 to 4, **characterized in that** said two pressure plates (3, 4) are movable relative to one another about a common pivot bearing (A).

6. Monitoring device according to claim 5, **characterized in that** said two pressure plates (3, 4), by means of the common pivot bearing (A), are designed such as to commonly accommodate the annular-shaped spring washer (5).

7. Monitoring device according to any one of claims 1 to 6, **characterized in that** different maximum loads of the snap element (6) are accounted for by adapted different spring strength or spring characteristic of individual annular-shaped spring washers (5).

8. Monitoring device according to any one of claims 1 to 6, **characterized in that** different maximum loads are accounted for by stacked multiple arrangement of annular-shaped spring washers (5), all of them having identical spring strength or spring characteristic of their snap element (6).

## Revendications

1. Dispositif de contrôle (1) à surveiller la charge de pression appliquée sur le pied durant la marche, qui est exercée par le poids du corps, moyennant un capteur de pression qui est placé en dessous du talon du pied à surveiller pour un indicateur de contrôle, audit capteur de pression étant configuré à un ressort en rondelle (5), qui renferme une partie à déclic (6) qui saut brusquement, d'une manière audible et tactile au talon, d'une position initiale en un état de surveillance sous la charge de pression maximale prédéterminée du pied et qui revient, de la manière d'un ressort, en la position initiale en réponse à une décompression du pied,
**caractérisé en ce que** ledit ressort en rondelle (5) a une configuration en anneau de cercle et que ladite partie à déclic et formée comme une bosse conique (6), qui entoure le trou central (7) dudit ressort en rondelle (5) et cause la partie en anneau adjacente dudit ressort en rondelle (5) à sauter brusquement en l'état de surveillance, et **en ce que** le dispositif de contrôle comprend deux plaques d'appui (3, 4) sous forme de semelle intérieure, entre lesquelles ledit ressort en rondelle en anneau de cercle est disposé.

2. Dispositif de contrôle selon la revendication 1, **caractérisé en ce que** l'une (3) desdites plaques d'appui est utilisée en tant qu'un appui du talon et comprend une zone en relief (9) formée pour l'appui contre ladite partie à déclic (6) dudit ressort en rondelle (5) en anneau de cercle.

3. Dispositif de contrôle selon la revendication 2, **caractérisé en ce que** ledit ressort en rondelle (5) en anneau de cercle est disposé au-dedans d'un creux (8) dans ladite deuxième plaque d'appui (4) en alignement sur ladite zone en relief (9) de ladite première plaque d'appui (3), en limitant l'état de surveillance en déclic de ladite partie à déclic (6) dudit ressort en rondelle (5) par ledit creux (8).

4. Dispositif de contrôle selon la revendication 2 ou 3, **caractérisé en ce que** lesdites deux plaques d'appui (3, 4) ont une configuration en coque de la façon d'un lit de talon.

5. Dispositif de contrôle selon une quelconque des revendications 2 à 4, **caractérisé en ce que** lesdites deux plaque d'appui (3, 4) sont mobiles, l'une relativement à l'autre, autour d'un palier de pivotement commun (A).

6. Dispositif de contrôle selon la revendication 5, **caractérisé en ce que** lesdites deux plaques d'appui (3, 4) sont formée, par ledit palier de pivotement commun (A), à recevoir, en commun, ledit ressort en rondelle (5) en anneau de cercle.

7. Dispositif de contrôle selon une quelconque des revendications 1 à 6, **caractérisé en ce que** des charges de pression maximales différentes de ladite partie à déclic (6) sont prises en considération par une dureté de ressort ou respectivement une caractéristique de flexibilité différente adaptée des ressorts en rondelle (5) individuels en anneau de cercle.

8. Dispositif de contrôle selon une quelconque des revendications 1 à 6, **caractérisé en ce que** des charges de pression maximales différentes sont prise en considération par un arrangement multiple stratifié des ressort en rondelle (5) en anneau de cercle, dont tous les ressort présentent la même dureté de ressort ou la même caractéristique de flexibilité de leur partie à déclic (6).
